(19) Europäisches Patentamt
European Patent Office
Office européen des brevets

(11) Veröffentlichungsnummer: **0 038 423**
**B1**

(12) # EUROPÄISCHE PATENTSCHRIFT

(45) Veröffentlichungstag der Patentschrift: **21.11.84**

(51) Int. Cl.³: **C 07 D 243/16** // C07F9/40

(21) Anmeldenummer: **81102038.7**

(22) Anmeldetag: **19.03.81**

(54) **Verfahren zur Herstellung von Benzodiazepin-Derivaten.**

(30) Priorität: **21.04.80 US 142581**

(43) Veröffentlichungstag der Anmeldung: **28.10.81 Patentblatt 81/43**

(45) Bekanntmachung des Hinweises auf die Patenterteilung: **21.11.84 Patentblatt 84/47**

(84) Benannte Vertragsstaaten: **AT BE CH DE FR GB IT LI NL**

(56) Entgegenhaltungen:
**BE - A - 833 248**

**S. PATAI: "The Chemistry of the carbonyl group", ed. Interscience publ. 1966, Seite 580, London, GB**
**G.M.KOSOLAPOFF: "Organic phosporus compounds", Band 3, Ausg. Wiley-Interscience "Phosphine alkylenes and phosphorus ylids. II. Phosphonate carbanions"**
**ORGANIC CHEMISTRY, Band 30, Nr. 12, Dezember 1965, Seiten 4276-4280, J.E. THOMPSON: "Enol and acyl phosphates as intermediates in the synthesis of nonrandom triglycerides"**
**ZVI RAPPOPORT: "The chemistry of the cyano group", 1970, Ausg. John Wiley & Sons, Seiten 589-590, London, G.B., Interscience Publishers "9. Cyanocarbon and polycyano compounds. XI. Cyanocarbon anions"**
**TETRAHEDRON LETTERS, No. 15, 1161-1162 (1969)**

(73) Patentinhaber: **F. HOFFMANN-LA ROCHE & CO. Aktiengesellschaft, CH-4002 Basel (CH)**

(72) Erfinder: **Niemczyk, Henry J., 50 Wilson Avenue, Wayne, N.J. 07450 (US)**

(74) Vertreter: **Kellenberger, Marcus, Dr. et al, Grenzacherstrasse 124 Postfach 3255, CH-4002 Basel (CH)**

(56) Entgegenhaltungen: (Fortsetzung)

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 038 423 B1

BUNDESDRUCKEREI BERLIN

**Beschreibung**

Die vorliegende Erfindung betrifft ein Verfahren zur Herstellung von Benzodiazepinderivaten der allgemeinen Formel

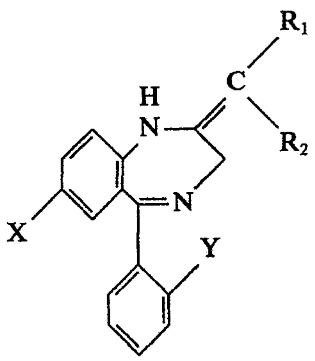

(I)

worin $R_1$ und $R_2$ Äthoxycarbonyl, Methoxycarbonyl oder Cyan bedeuten, mit der Auflage, daß $R_1$ und $R_2$ nicht beide Cyan bedeuten können, Y Wasserstoff oder Halogen und X Wasserstoff, Halogen oder Nitro bedeuten,
gemäß welchem man ein Benzodiazepin-2-on der allgemeinen Formel

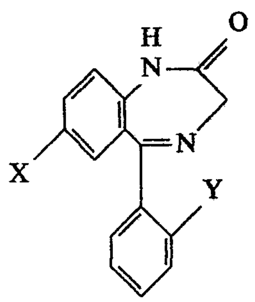

(III)

worin X und Y die oben angegebene Bedeutung besitzen, mit einem Phosphonatanion der allgemeinen Formel

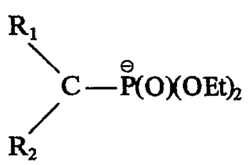

(II)

worin $R_1$ und $R_2$ die oben angegebene Bedeutung besitzen,
umsetzt.

Die Verbindungen der Formel I sind wertvolle Zwischenprodukte zur Herstellung von Imidazobenzodiazepinen, welche bekannte Sedativa, Muskelrelaxantien, Antikonvulsiva und Anxiolytika sind. Wie die Verbindungen der obigen Formel I in diese Imidazobenzodiazepine übergeführt werden können, ist beispielsweise in der belgischen Patentschrift No. 833 248 beschrieben.

Aus Tetrahedron Letters, 15, 1161–1162 (1969) ist es bekannt, daß Phthalimide und Succinimide mit Hilfe von Triphenylphosphinäthoxycarbonylmethylen in einer Wittig-Reaktion in die entsprechenden Monoalkyliden-Verbindungen übergeführt werden können. Aus S. Patai, The Chemistry of the Carbonyl Group, Interscience Publ. 1966, ist bekannt, daß man die Wittig-Reagenzien, d. h. die Yliden-Phosphorane, durch die Wittig-Horner-Reagenzien, nämlich Phosphonat-Anionen, ersetzen kann.

Der in dieser Beschreibung verwendete Ausdruck »Halogen« betrifft die vier Formen Chlor, Fluor, Brom und Jod.

Das Verfahren gemäß vorliegender Erfindung zur Herstellung der Verbindungen der obigen Formel I ist eigentlich ein Zweistufenverfahren, wobei das Phosphonatanion der Formel II in situ gebildet wird. Im nachstehenden Reaktionsschema wird dieses Verfahren illustriert:

Alkalimetallhydrid + (A) Malonsäurediäthylester + Diäthylchlorphosphat

oder

(B) Cyanessigsäureäthylester

oder

(C) Malonsäuredimethylester

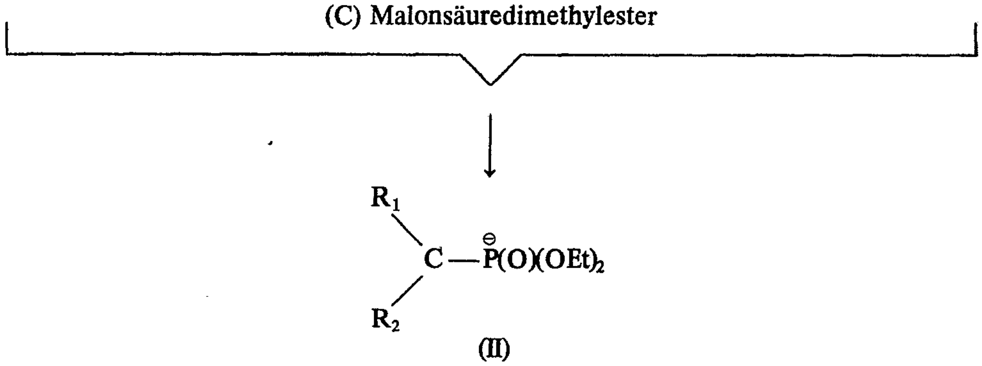

(II)

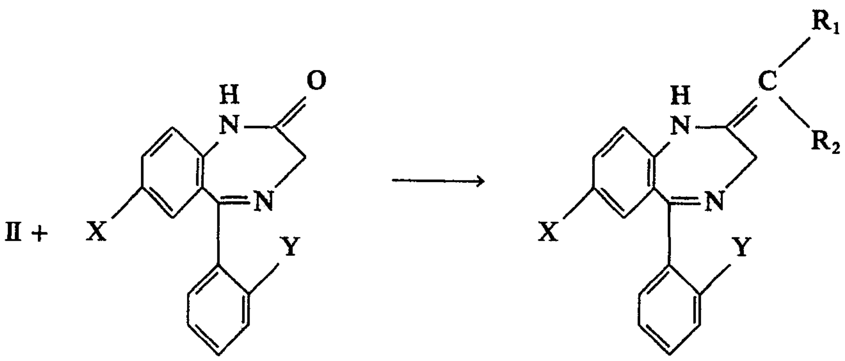


(III) (I)

worin $R_1$, $R_2$, Y und X die oben angegebene Bedeutung besitzen.

Die Verbindungen der Formel I werden hergestellt, indem man Malonsäurediäthylester, Malonsäuredimethylester oder Cyanessigsäureäthylester in einem inerten Äther, wie Tetrahydrofuran oder Dioxan, oder einem anderen inerten Lösungsmittel, wie Dimethylformamid oder Methylenchlorid, in Gegenwart eines Alkalimetallhydrides, beispielsweise Natriumhydrid (zur Bildung des entsprechenden Anions), mit Diäthylchlorphosphat unter Bildung des Phosphonatanions der Formel II umsetzt und danach das Phosphonatanion der Formel II in situ mit einem Benzodiazepinderivat der Formel III zur Reaktion bringt. Für beide Reaktionsschritte können die gleichen Lösungsmittel verwendet werden, und die Reaktionstemperatur liegt für beide Schritte zwischen etwa 0°C und der Rückflußtemperatur des verwendeten Lösungsmittels, vorzugsweise bei ungefähr Raumtemperatur.

Die folgenden Beispiele illustrieren die Erfindung, ohne diese zu beschränken. Alle Temperaturen sind in Celsiusgraden angegeben.

## Beispiel 1

91 ml (0,6 Mol) Malonsäurediäthylester werden zu 300 ml Tetrahydrofuran gegeben. Das Reaktionsgemisch wird mit einem Eisbad auf Raumtemperatur gehalten, während man innerhalb einer Stunde 32 g Natriumhydrid (60 %ige Dispersion in Öl) zugibt. Zur erhaltenen Suspension werden innerhalb von 50 Minuten 29 ml Diäthylchlorphosphat gegeben. Nach 30minütigem Schütteln wird eine Lösung von 28,85 g 7-Chlor-5-(2-fluorphenyl)-1,3-dihydro-2 H-1,4-benzodiazepin-2-on in Tetrahydrofuran innerhalb von 20 Minuten zugetropft. Nach beendeter Zugabe wird die erhaltene Lösung während 2 Stunden geschüttelt, und danach wird der pH-Wert auf 5,0 bis 5,5 eingestellt. Der größte Teil des Tetrahydrofuran wird unter vermindertem Druck (40°/4000 Pa) entfernt, und dann werden zuerst 600 ml kaltes Wasser und dann 100 ml Hexan zugegeben. Die erhaltene Suspension wird filtriert und mit Wasser und Hexan gewaschen. Nach dem Trocknen erhält man als Endprodukt 7-Chlor-5-(2-fluorphenyl)-1,3-dihydro-2 H-1,4-benzodiazepin-2-yliden-malonsäurediäthylester.

## Beispiel 2

45,55 ml Malonsäurediäthylester werden zu 200 ml Tetrahydrofuran gegeben. Das Reaktionsgemisch wird bei Raumtemperatur gehalten, während 16 g Natriumhydrid (60 %ige Dispersion in Öl) innerhalb von einer Stunde zugegeben werden. 14,45 ml Diäthylchlorphosphat werden tropfenweise innerhalb von 50 Minuten zur erhaltenen Suspension gegeben. Nach 30minütigem Schütteln und Rühren wird eine Lösung von 13,54 g 7-Chlor-1,3-dihydro-5-phenyl-2 H-1,4-benzodiazepin-2-on in Tetrahydrofuran innerhalb von 20 Minuten tropfenweise zugegeben. Die erhaltene Lösung wird während einer Stunde geschüttelt. Danach wird der pH-Wert mit Essigsäure auf 5,0 bis 5,5 eingestellt, und die Reaktionslösung unter vermindertem Druck (40°/4000 Pa) zur Entfernung des Tetrahydrofuran eingeengt. Zum Rückstand werden 600 ml kaltes Wasser und 100 ml Hexan gegeben. Der dabei gebildete kristalline Festkörper wird abfiltriert und mit Wasser und Hexan gewaschen. Nach dem Trocknen des Festkörpers erhält man 7-Chlor-1,3-dihydro-5-phenyl-2 H-1,4-benzodiazepin-2-yliden-malonsäurediäthylester als Endprodukt.

## Beispiel 3

45,55 ml Malonsäurediäthylester werden zu 200 ml Tetrahydrofuran gegeben. Das Reaktionsgemisch wird mit einem Eisbad auf Raumtemperatur gehalten, während man 14 g (60 %ige Dispersion in Öl) Natriumhydrid portionenweise innerhalb einer Stunde zugibt. Die erhaltene Suspension wird tropfenweise innerhalb von 50 Minuten mit 14,45 ml Diäthylchlorphosphat versetzt. Nach 30minütigem Schütteln wird eine Lösung von 15,25 g 7-Chlor-5-(2-chlorphenyl)-1,3-dihydro-2 H-1,4-benzodiazepin-2-on in Tetrahydrofuran innerhalb von 20 Minuten zugetropft. Die erhaltene Lösung wird während einer Stunde geschüttelt. Der pH-Wert wird danach auf 5,0 bis 5,5 eingestellt, und die Reaktionslösung zur Entfernung des Tetrahydrofuran bei 40°/4000 Pa eingeengt. Zum erhaltenen Rückstand werden 600 ml kaltes Wasser und 100 ml Hexan gegeben. Der erhaltene kristalline Festkörper wird abfiltriert und mit kaltem Wasser und Hexan gewaschen. Nach dem Trocknen des Festkörpers bis zur Gewichtskonstanz erhält man 7-Chlor-5-(2-chlorphenyl)-1,3-dihydro-2 H-1,4-benzodiazepin-2-yliden-malonsäurediäthylester als Endprodukt.

## Beispiel 4

45,55 ml Malonsäurediäthylester werden zu 200 ml Tetrahydrofuran gegeben. Das Reaktionsgemisch wird mit einem Eisbad auf Raumtemperatur gehalten, während man 16 g (60 %ige Dispersion in Öl) Natriumhydrid portionsweise innerhalb einer Stunde zugibt. Zur erhaltenen Suspension werden innerhalb von 50 Minuten 14,45 ml Diäthylchlorphosphat getropft. Nach 30minütigem Schütteln wird eine Lösung von 15,8 g 5-(2-Chlorphenyl)-7-nitro-2 H-1,4-benzodiazepin-2-on in Tetrahydrofuran innerhalb von 20 Minuten tropfenweise zugegeben. Die erhaltene Lösung wird während einer Stunde geschüttelt. Danach wird der pH-Wert auf 5,0 bis 5,5 eingestellt, und die Reaktionslösung zur Entfernung des Tetrahydrofuran bei 40°/4000 Pa eingeengt. Zum Rückstand werden 600 ml kaltes Wasser und 100 ml Hexan gegeben. Der erhaltene kristalline Festkörper wird abfiltriert und mit kaltem Wasser und Hexan gewaschen. Nach dem Trocknen des Festkörpers bis zur Gewichtskonstanz erhält man 5-(2-Chlorphenyl)-1,3-dihydro-7-nitro-2 H-1,4-benzodiazepin-2-yliden-malonsäurediäthylester als Endprodukt.

## Beispiel 5

32,13 ml Cyanessigsäureäthylester werden zu 200 ml Tetrahydrofuran gegeben. Danach werden innerhalb einer Stunde 16 g Natriumhydrid in Portionen zugegeben. Zur erhaltenen Suspension werden 14,45 ml Diäthylchlorphosphat gegeben. Nach 30minütigem Schütteln wird eine Lösung von 14,43 g 7-Chlor-5-(2-fluorphenyl)-1,3-dihydro-2 H-1,4-benzodiazepin-2-on in Tetrahydrofuran innerhalb einer Stunde zugetropft. Nach einstündigem Schütteln wird die Reaktionslösung mit Essigsäure auf pH 5 eingestellt und mit 500 ml kaltem Wasser und 300 ml Hexan versetzt. Die erhaltene Suspension von Kristallen wird filtriert, und der Rückstand mit Wasser und Hexan gewaschen. Nach dem Trocknen der Kristalle bis zur Gewichtskonstanz erhält man 7-Chlor-5-(2-fluorphenyl)-1,3-dihydro-2 H-1,4-benzodiazepin-2-yliden-cyanessigsäureäthylester als Endprodukt.

**Patentansprüche**

1. Verfahren zur Herstellung von Benzodiazepinderivaten der allgemeinen Formel

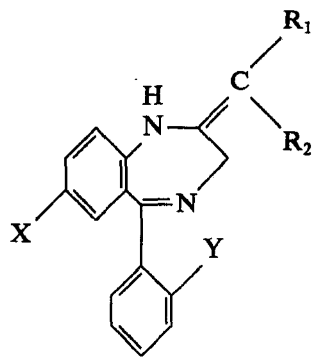

(I)

worin $R_1$ und $R_2$ Äthoxycarbonyl, Methoxycarbonyl oder Cyan bedeuten, mit der Auflage, daß $R_1$ und $R_2$ nicht beide Cyan bedeuten können, Y Wasserstoff oder Halogen und X Wasserstoff, Halogen oder Nitro bedeuten,
dadurch gekennzeichnet, daß man ein Benzodiazepin-2-on der allgemeinen Formel

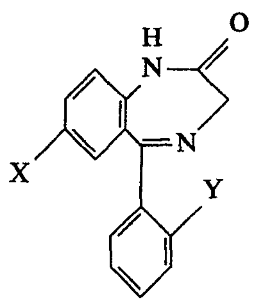

(III)

worin X und Y die oben angegebene Bedeutung besitzen, mit einem Phosphonatanion der allgemeinen Formel

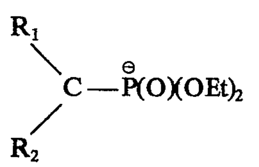

(II)

worin $R_1$ und $R_2$ die oben angegebene Bedeutung besitzen,
umsetzt.

2. Verfahren nach Anspruch 1, dadurch gekennzeichnet, daß man das Phosphonatanion der Formel II so herstellt, indem man ein Alkalimetallhydrid mit Malonsäurediäthylester, Malonsäuredimethylester oder Cyanessigsäureäthylester unter Bildung des entsprechenden Anions umsetzt und dieses danach mit Diäthylchlorphosphat zur Reaktion bringt.

3. Verfahren nach Anspruch 2, dadurch gekennzeichnet, daß man als Alkalimetallhydrid Natriumhydrid verwendet.

4. Verfahren nach Anspruch 2 oder 3, dadurch gekennzeichnet, daß man das Hydrid mit Malonsäurediäthylester umsetzt.

5. Verfahren nach einem der Ansprüche 1–4, dadurch gekennzeichnet, daß man die Reaktion bei Raumtemperatur durchführt.

6. Verfahren nach einem der Ansprüche 1–5, dadurch gekennzeichnet, daß man die Reaktion in Tetrahydrofuran durchführt.

**Claims**

1. A process for the manufacture of benzodiazepine derivatives of the general formula

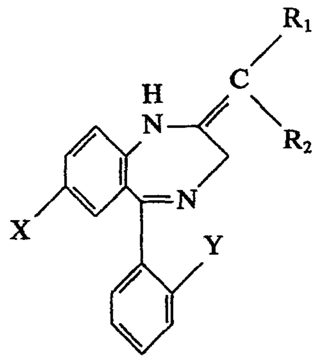

(I)

wherein $R_1$ and $R_2$ signify ethoxycarbonyl, methoxycarbonyl or cyano, with the proviso that $R_1$ and $R_2$ can not both signify cyano, Y signifies hydrogen or halogen and X signifies hydrogen, halogen or nitro, characterized by reacting a benzodiazepin-2-one of the general formula

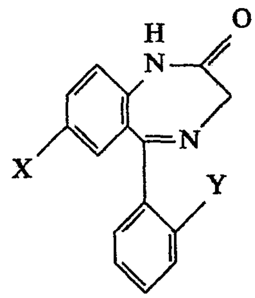

(III)

wherein X and Y have the significance given above, with a phosphonate anion of the general formula

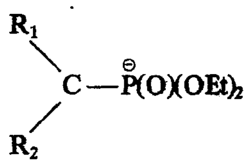

(II)

wherein $R_1$ and $R_2$ have the significance given above.

2. A process according to claim 1, characterized in that the phosphonate anion of formula II is prepared by reacting an alkali metal hydride with malonic acid diethyl ester, malonic acid dimethyl ester or cyanoacetic acid ethyl ester with the formation of the corresponding anion and thereafter reacting this with diethyl chlorophosphonate.

3. A process according to claim 2, characterized in that sodium hydride is used as the alkali metal hydride.

4. A process according to claim 2 or 3, characterized in that the hydride is reacted with malonic acid diethyl ester.

5. A process according to any one of claims 1—4, characterized in that the reaction is carried out at room temperature.

6. A process according to any one of claims 1—5, characterized in that the reaction is carried out in tetrahydrofuran.

**Revendications**

1. Procédé de préparation de dérivés de la benzodiazépine répondant à la formule générale

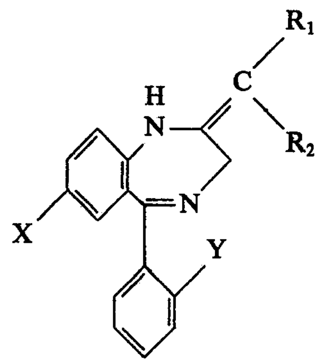

(I)

dans laquelle $R_1$ et $R_2$ représentent des groupes éthoxycarbonyle, méthoxycarbonyle ou cyano, étant spécifié que $R_1$ et $R_2$ ne peuvent représenter tous deux des groupes cyano, Y représente l'hydrogène ou un halogène et X représente l'hydrogène, un halogène ou un groupe nitro,
charactérisé en ce que l'on fait réagir une benzodiazépine-2-one de formule générale

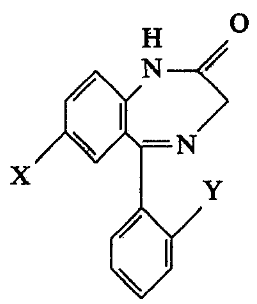

(III)

dans laquelle X et Y ont les significations indiquées ci-dessus,
avec un anion phosphonate de formule générale

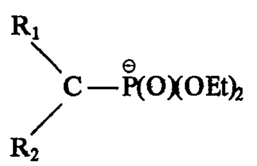

(II)

dans laquelle $R_1$ et $R_2$ ont les significations indiquées ci-dessus.

2. Procédé selon la revendication 1, caractérisé en ce que l'on prépare l'anion phosphonate de formule II en faisant réagir un hydrure de métal alcalin avec du malonate de diéthyle, du malonate de diméthyle ou du cyanacétate d'éthyle, avec formation de l'anion correspondant qu'on fait ensuite réagir avec du chlorophosphate de diéthyle.

3. Procédé selon la revendication 2, caractérisé en ce que l'hydrure de métal alcalin utilisé est l'hydrure de sodium.

4. Procédé selon la revendication 2 ou 3, caractérisé en ce que l'on fait réagir l'hydrure avec le malonate de diéthyle.

5. Procédé selon l'une des revendications 1 à 4, caractérisé en ce que la réaction est effectuée à température ambiante.

6. Procédé selon l'une des revendications 1 à 5, caractérisé en ce que la réaction est effectuée dans le tétrahydrofuranne.